# EUROPÄISCHE PATENTSCHRIFT

(11) **EP 3 334 975 B1**
(45) Veröffentlichungstag und Bekanntmachung des Hinweises auf die Patenterteilung: **31.07.2019**
(21) Anmeldenummer: 16754457.6
(22) Anmeldetag: 08.08.2016
(51) Int. Cl.: F21V 23/04, H05B 33/08, H05B 37/02, A61B 90/30, F21Y 115/10, F21W 131/202, F21W 131/205

(54) **OPERATIONSLEUCHTE MIT HELLIGKEITSREGULIERUNG**
SURGICAL LAMP WITH BRIGHTNESS REGULATION
LAMPE DE SALLE D'OPÉRATION AVEC RÉGULATION DE LUMINOSITÉ

(30) Priorität: 13.08.2015 DE 102015113339
(43) Veröffentlichungstag der Anmeldung: 20.06.2018
(73) Patentinhaber: Karl Leibinger Medizintechnik GmbH & Co. KG, 78570 Mühlheim/Donau (DE)
(72) Erfinder: STRÖLIN, Joachim, 78604 Rietheim (DE)
(74) Vertreter: Winter, Brandl, Fürniss, Hübner, Röss, Kaiser, Polte - Partnerschaft mbB
(86) Internationale Anmeldenummer: PCT/EP2016/068893
(87) Internationale Veröffentlichungsnummer: WO 2017/025513

(56) Entgegenhaltungen:
- EP-A1- 2 136 126
- EP-A1- 2 136 128
- EP-A1- 2 434 202
- DE-A1-102013 012 231
- US-A- 5 068 767

## Beschreibung

Die Erfindung betrifft eine Operationsleuchte zum Ausleuchten eines Operationsfeldes, mit einer Vielzahl an Einzelleuchten, die in einem eingeschalteten Zustand jeweils ein sich entlang einer Längsachse erstreckendes sowie in einer Beleuchtungsebene einen Lichtfeldbereich erzeugendes Lichtstrahlbündel ausbilden, wobei die Lichtfeldbereiche der Einzelleuchten in der Beleuchtungsebene so nebeneinander und/oder zumindest teilweise übereinander (d.h. sich teilweise überdeckend) angeordnet sind, dass ein Gesamtlichtfeld (der Operationsleuchte) gebildet ist, mit einer Helligkeitserfassungseinrichtung, die ausgebildet ist, einen Ist-Helligkeitswert in dem Gesamtlichtfeld zu ermitteln, sowie mit einer Steuereinheit, die in Abhängigkeit des durch die Helligkeitserfassungseinrichtung (in einem Messbereich innerhalb des Gesamtlichtfeldes) ermittelten Ist-Helligkeitswertes beleuchtungsstärkesteuernd auf die Einzelleuchten einwirkt.

Aus dem Stand der Technik sind bereits gattungsgemäße Operationsleuchten bekannt. Beispielsweise offenbart die US 8,710,415 B2 eine Beleuchtungsvorrichtung mit einer Helligkeitsregulierungsvorrichtung zur Helligkeitsregulierung in Abhängigkeit der Helligkeit eines beleuchteten Feldes. EP 2 136 126, EP 2 136 128 und DE 10 2013 012231 ferner offenbaren Operationsleuchten des Stands der Technik.

Bei den aus dem Stand der Technik bekannten Operationsleuchten hat es sich jedoch als nachteilig herausgestellt, dass deren Gesamtlichtfelder, wenn eine automatische Helligkeitssteuerung vorhanden ist, nur relativ schwierig individuelle auf den jeweiligen Operationsbereich an einem zu operierenden Körper einstellbar sind. Dies bringt insbesondere den Nachteil mit sich, dass während der Operation auch Bereiche eines zu operierenden Körpers erhellt werden, die eigentlich nicht mit erhellt werden sollten.

Dies sind bspw. hellere / stärker reflektierende Bereiche des Körpers, wie z.B. Hautbereiche, Knochen oder Fettgewebe. Durch Erhellen dieser Bereiche (aufgrund einer Erhöhung der Beleuchtungsstärke) kommt es häufig zu einem Blenden des Operateurs. Für andere, dunklere Bereiche, etwa stärker durchblutete oder dunklere Organe des Körpers, die viel Licht absorbieren und daher relativ wenig Lichtstrahlen reflektieren, ist es zudem notwendig eine im Vergleich zu den helleren Bereichen stärkere Beleuchtung / höhere Beleuchtungsstärke zu ermöglichen. Denn diese dunklen Bereiche sind häufig relativ schwierig von dem Operateur zu erkennen. Somit besteht mit den Operationsleuchten des Standes der Technik häufig das Problem, dass der Operationsbereich lokal entweder zu hell oder zu dunkel eingestellt ist, wodurch es sein kann, dass der Operateur den Operationsbereich des Körpers stellenweise nicht richtig erkennen kann oder stark geblendet wird. Auch ist es möglich, dass bei einer zu starken Beleuchtung des Gesamtlichtfeldes Bereiche des zu operierenden Körpers relativ rasch austrocknen.

Es ist daher die Aufgabe der vorliegenden Erfindung, die aus dem Stand der Technik bekannten Nachteile zu beheben und insbesondere eine Operationsleuchte zur Verfügung zu stellen, die möglichst variabel auf lokale Verhältnisse in einem Operationsbereich hinsichtlich ihrer Ausleuchtung einstellbar sein soll.

Dies wird erfindungsgemäß durch eine Operationsleuchte mit den Merkmalen des Anspruchs 1 gelöst.

Dies wird erfindungsgemäß dadurch gelöst, dass die Helligkeitserfassungseinrichtung sowie die Steuereinheit derart ausgebildet sind und die Steuereinheit derart mit den Einzelleuchten verbunden ist, dass abhängig von dem Ist-Helligkeitswert eine Beleuchtungsstärke einer ersten Einzelleuchte gezielt und unabhängig von einer Beleuchtungsstärke einer zweiten Einzelleuchte zum Erhellen oder Verdunkeln eines (durch die erste Einzelleuchte ausgebildeten) ersten Lichtfeldbereiches einstellbar ist. Dadurch ist es ermöglicht, dass Einzelleuchten getrennt / unabhängig voneinander in ihrer Beleuchtungsstärke steuerbar sind, wodurch das Gesamtlichtfeld der Operationsleuchte in den einzelnen Lichtfeldbereichen unterschiedlich stark erhellt oder verdunkelt werden kann. Dadurch können insbesondere nur die Bereiche des Gesamtlichtfeldes in ihrer Helligkeit angepasst werden, für die eine solche Erhellung sinnvoll ist. Beispielsweise stark reflektierende Bereiche können somit weniger stark oder nicht miterhellt werden, wohingegen dunkle, viel Licht absorbierende Bereiche deutlich erhellt werden können. Dies ermöglicht dem Operateur, eine deutlichere Sichtverbesserung des Operationsbereiches.

Weitere vorteilhafte Ausführungsformen sind in den Unteransprüchen beansprucht und nachfolgend näher erläutert.

Demnach ist es besonders von Vorteil, wenn die Einzelleuchten in mehrere Leuchtengruppen aufgeteilt sind, wobei mehrere einer ersten Leuchtengruppe zugeordnete (erste) Einzelleuchten derart ausgerichtet und zueinander angeordnet sind, dass sich die Längsachsen ihrer Lichtstrahlbündel in (einem gemeinsamen ersten Schnittpunkt) in einer ersten gemeinsamen Fokusebene schneiden.

In diesem Zusammenhang ist es weiter vorteilhaft, wenn dann mehrere einer zweiten Leuchtengruppe zugeordnete (zweite) Einzelleuchten derart ausgerichtet und zueinander angeordnet sind, dass sich die Längsachsen ihrer Lichtstrahlbündel in (einem gemeinsamen (zweiten) Schnittpunkt) in einer, zu der ersten Fokusebene beabstandet angeordneten, zweiten Fokusebene schneiden. Dadurch ist durch die Operationsleuchte auch eine besonders gute Tiefenausleuchtung gegeben.

Ist die Steuereinheit elektrisch und / oder datenübermittelnd / -übertragend mit den (ersten) Einzelleuchten der ersten Leuchtengruppe und / oder den (zweiten) Einzelleuchten der zweiten Leuchtengruppe verbunden, können die Einzelleuchten innerhalb der Leuchtengruppe ebenfalls unabhängig voneinander beleuchtungsstärkeeinstellend angesteuert werden. Auch können die Einzelleuchten der unterschiedlichen Gruppen unabhängig voneinander angesteuert werden. Dadurch ist eine besonders vielfältige Beleuchtungsstärkesteuerung / Helligkeitssteuerung umgesetzt, um die unterschiedlichen, individuellen Operationsbereiche stets ausreichend stark, jedoch nicht zu stark zu erhellen.

Zweckmäßig ist es auch, wenn die Helligkeitserfassungseinrichtung zumindest einen Helligkeitssensor umfasst, der zumindest einen Fototransistor, zumindest einen Fotowiderstand und/oder zumindest eine Fotodiode aufweist. Dadurch ist die Helligkeitserfassungseinrichtung auch besonders kostengünstig ausführbar.

In diesem Zusammenhang ist es besonders vorteilhaft, wenn der zumindest eine Helligkeitssensor Teil einer (vorzugsweise medizinischen) Kamera (Foto / Videokamera) der Helligkeitserfassungseinrichtung ist. Denn dadurch ist es besonders effizient möglich, die Helligkeit in einem Messbereich der Helligkeitserfassungseinrichtung zu ermitteln.

Erfasst die Helligkeitserfassungseinrichtung zudem zumindest einen Messbereich innerhalb des Gesamtlichtfeldes, welcher Messbereich eine kleinere Fläche aufweist als das Gesamtlichtfeld, ist es stets vermieden, dass die Helligkeitserfassungseinrichtung nicht unerwünschte Bereiche außerhalb des relevanten Operationsbereiches mit erfasst.

In diesem Zusammenhang ist es auch besonders vorteilhaft, wenn die Helligkeitserfassungseinrichtung den Ist-Helligkeitswert des Messbereiches gesamtheitlich (d.h. integral) oder ausschnittsweise / abschnittsweise ("Spot" / punktweise) ermittelt. Dadurch lassen sich die Ist-Helligkeitswerte besonders verlässlich ermitteln.

Dabei ist es weiterhin vorteilhaft, wenn die Einzelleuchten im Betrieb der Operationsleuchte, d.h. in ihrem eingeschalteten Zustand, jeweils derartige Lichtfeldbereiche ausbilden, dass sich diese stets um einen gewissen Flächenanteil mit zumindest einem weiteren / benachbarten Lichtfeldbereich überlappen / überdecken und sich ein Gesamtlichtfeld ergibt, das in einer Ebene eine zusammenhängende Fläche einnimmt / ausbildet. Dadurch ist eine besonders effiziente Ausleuchtung umgesetzt.

Erfindungsgemäß weist die Helligkeitserfassungseinrichtung weiterhin eine Kamera auf, wodurch die Helligkeitserfassungseinrichtung besonders kostengünstig ausgestaltet werden kann.

Erfindungsgemäß weist die Kamera ein Objektiv mit einer festes / fest eingestellten Brennweite auf. Die Brennweite ist beispielsweise derart gewählt, dass das Gesamtlichtfeld in einer Ebene des Brennpunktes des Objektivs befindlich ist. In diesem Zusammenhang ist es auch besonders vorteilhaft, wenn das Gesamtlichtfeld in einer Ebene des Brennpunktes des Objektivs größer als ein optisch durch die Kamera erfasster Messbereich in dieser Ebene ist. Dadurch ist eine Erfassung der Helligkeit besonders effektiv durchführbar, wobei die Helligkeitsmessung auch bei stark erhellten Zuständen / Bereichen umsetzbar ist, da lediglich die Belichtungszeit angepasst werden muss.

Um die Steuerung der Kamera weiter zu erleichtern, ist in einer weiteren Ausführung ein Blendenwert (d.h. eine Lichtdurchlassöffnung des Objektivs, die zum Sensor / Helligkeitssensor der Kamera führt) der Kamera festgelegt / fest eingestellt (insbesondere wenn ein Belichtungszeitwert der Kamera einstellbar ist). Alternativ ist dieser Blendenwert der Kamera einstellbar / veränderbar (insbesondere wenn ein Belichtungszeitwert der Kamera festgelegt / fest eingestellt ist). Dadurch ist die Helligkeitsmessung besonders einfach automatisierbar.

Somit ist es auch zweckdienlich, wenn ein Belichtungszeitwert der Kamera fest eingestellt ist oder einstellbar ist, wodurch ebenfalls eine Helligkeitsmessung effektiver in der Durchführung ist.

Umfasst die Helligkeitserfassungseinrichtung ein Leuchtdichtemessgerät oder ist die Helligkeitserfassungseinrichtung als ein solches Leuchtdichtemessgerät ausgebildet, wobei dann vorzugsweise die zuvor beschriebene Kamera als ein Leuchtdichtemessgerät ausgebildet ist, ist die Helligkeit besonders präzise feststellbar.

Im Weiteren ist es auch zweckmäßig, wenn die Helligkeitserfassungseinrichtung in einem die Einzelleuchten aufnehmenden Leuchtenaufnahmekörper eingesetzt / eingebaut / positioniert / integriert ist oder in einer mit dem Leuchtenaufnahmekörper wieder abnehmbar verbindbaren Handgriffvorrichtung eingesetzt / eingebaut / positioniert / integriert ist. Dadurch wird kein zusätzlicher Bauraum für die Helligkeitserfassungseinrichtung benötigt und die Operationsleuchte ist im Wesentlichen ohne zusätzlichen Bauraum ausbildbar.

In diesem Zusammenhang ist es besonders von Vorteil, wenn jede Einzelleuchte dadurch definiert ist / ausgebildet ist, dass sie eine einzelne Lampe, vorzugsweise eine LED-Lampe aufweist, die zusammen mit einer Linsenoptik in einer Leuchteneinheit / einem Leuchtenmodul integriert ist. Dadurch lassen sich die Einzelleuchten besonders einfach ansteuern.

Erfasst die Helligkeitserfassungseinrichtung den Ist-Helligkeitswert kontinuierlich / dauerhaft, intervallartig oder nach manueller Eingabe eines Aufnahmebefehls durch eine mit der Steuereinheit verbundenen Betätigungseinrichtung, ist die Anpassung der Helligkeit besonders variabel möglich.

Vorteilhafterweise ist die Helligkeitserfassungseinrichtung auch derart ausgebildet, dass sie Bereiche in einem bestimmten Abstand zum Leuchtenaufnahmekörper weg, etwa mindestens 50 cm von dem Leuchtenaufnahmekörper weg messtechnisch ausblendet. Dadurch ist stets gewährleistet, dass nicht versehentlich ein nicht die Beleuchtungsebene direkt störendes / bedeckendes Element fehlbeleuchtet wird.

Weiterhin betrifft die Erfindung auch ein Verfahren zur Steuerung einer solchen Operationsleuchte nach zumindest einer der zuvor beschriebenen Ausführungsformen zum Ausleuchten eines Operationsfeldes, wobei die Operationsleuchte eine Vielzahl an Einzelleuchten, die in einem eingeschalteten Zustand jeweils ein sich entlang einer Längsachse erstreckendes sowie in einer Beleuchtungsebene einen Lichtfeldbereich erzeugendes Lichtstrahlbündel ausbilden, wobei die Lichtfeldbereiche der Einzelleuchten in der Beleuchtungsebene so nebeneinander und/oder zumindest teilweise übereinander angeordnet sind, dass ein Gesamtlichtfeld gebildet ist; eine Helligkeitserfassungseinrichtung, die ausgebildet ist, einen Ist-Helligkeitswert in dem Gesamtlichtfeld zu ermitteln, sowie einer Steuereinheit, die in Abhängigkeit des ermittelten Ist-Helligkeitswertes beleuchtungsstärkesteuernd auf die Einzelleuchten einwirkt, aufweist, wobei die Helligkeitserfassungseinrichtung sowie die Steuereinheit derart ausgebildet sind und die Steuereinheit derart mit den Einzelleuchten verbunden ist, dass abhängig von dem Ist-Helligkeitswert eine Beleuchtungsstärke einer ersten Einzelleuchte gezielt und unabhängig von einer Beleuchtungsstärke einer zweiten Einzelleuchte zum Erhellen oder Verdunkeln eines ersten Lichtfeldbereiches eingestellt wird.

Die Erfindung wird nun nachfolgend anhand von Figuren näher erläutert, in welchem Zusammenhang auch verschiedene Ausführungsbeispiele beschrieben sind.

Es zeigen:

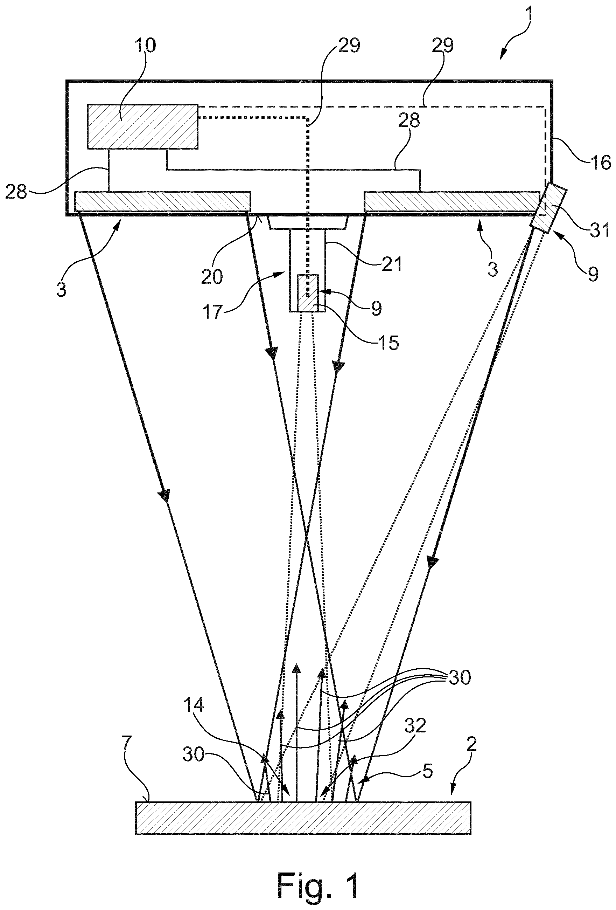

- Fig. 1: eine schematische Ansicht einer Operationsleuchte der erfindungsgemäßen Art, wobei ein Leuchtenaufnahmekörper der Operationsleuchte teilweise geschnitten dargestellt ist und eine Helligkeitserfassungseinrichtung der Operationsleuchte innerhalb einer Handgriffvorrichtung, die an dem Leuchtenaufnahmekörper der Operationsleuchte wiederabnehmbar befestigt ist, integriert ist, und

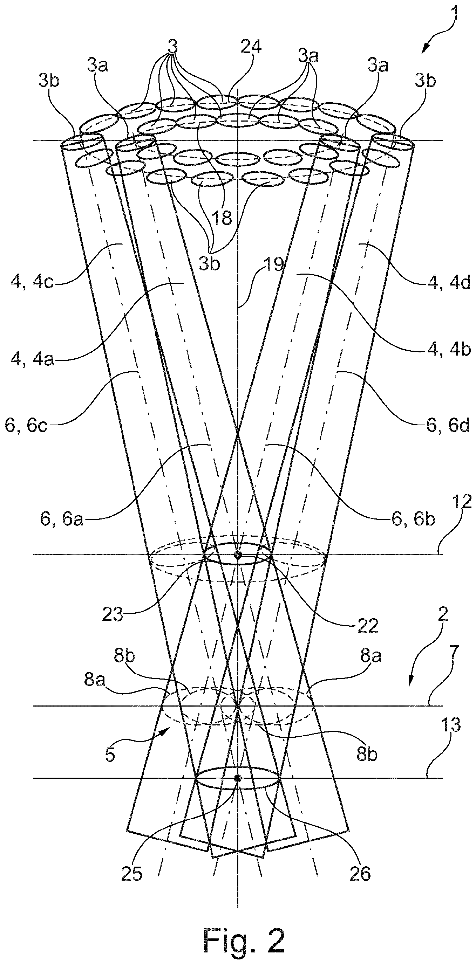

- Fig. 2: eine schematische Darstellung der Operationsleuchte nach Fig. 1, wobei besonders gut die Einteilung der Vielzahl in der Operationsleuchte enthaltenen Einzelleuchten in verschiedene Leuchtengruppen erkennbar ist.

Die Figuren sind lediglich schematischer Natur und dienen ausschließlich dem Verständnis der Erfindung. Die gleichen Elemente sind mit denselben Bezugszeichen versehen.

Die erfindungsgemäße Operationsleuchte 1 ist in Fig. 2 in ihrem schematischen Aufbau besonders gut zu erkennen. Die Operationsleuchte 1 dient hierbei auf übliche Weise dem Ausleuchten / Beleuchten eines Operationsfeldes 2 eines auf einem Behandlungstisch befindlichen Objekts, etwa einem Menschen.

Die Operationsleuchte 1 weist eine Vielzahl an einzelnen Leuchten auf, die nachfolgend als Einzelleuchten 3 bezeichnet sind. Jede Einzelleuchte 3 besteht dabei aus einem einzelnen, d.h. stofflich einzeln / separat von den übrigen Einzelleuchten 3 ausgeformten Leuchtenmodul. Das Leuchtenmodul umfasst wiederum eine LED-Lampe / -Birne / ein LED-Licht und eine zugehörigen Linse / Linsenoptik. Die Einzelleuchten 3 weisen dabei in ihrem Leuchtenmodul jeweils stets nur eine LED auf. Auch weist jedes Leuchtenmodul entsprechende Reflektoren bzw. Vorrichtungen zum Bündeln des durch die LED imitierten Lichts auf, welches Licht seitens der Linse aus dem Leuchtenmodul in Form eines Lichtstrahlbündels 4 austritt. Somit bildet jede Einzelleuchte 3 in einem eingeschalteten / bestromten Zustand ein sich entlang einer Längsachse 6 erstreckendes Lichtstrahlbündel 4 aus. In anderen Worten ausgedrückt, erzeugt jede Einzelleuchte 3 bzw. jedes Leuchtmodul der Einzelleuchte 3 ein Lichtstrahlbündel 4.

In Fig. 2 sind zur schematisch Darstellung der Ausrichtung der Einzelleuchten 3 zwei erste Einzelleuchten 3a, die einer ersten Leuchtengruppe 11a/Linsengruppe zugeordnet sind, angeschaltet. Auch sind zwei zweite Einzelleuchten 3b, die einer zweiten Leuchtengruppe 11b/Linsengruppe zugeordnet sind, angeschaltet.

Die erste Leuchtengruppe 11a besteht in dieser Ausführung nicht nur aus zwei, sondern aus mehr als zwei ersten Einzelleuchten 3a. Insgesamt sind in der ersten Leuchtengruppe 11a zwölf der ersten Einzelleuchten 3a enthalten. In weiteren Ausführungen beträgt die Anzahl der ersten Einzelleuchten 3a der ersten Leuchtengruppe 11a jedoch auch mehr als zwölf oder weniger als zwölf.

Die ersten Einzelleuchten 3a der ersten Leuchtengruppe 11a sind entlang einer ringförmigen / kreisringförmigen Umfangslinie, die nachfolgend als erste Umfangslinie 18 bezeichnet ist, angeordnet. Die erste Umfangslinie 18 ist dabei zentrisch zu einer gedachten Mittelachse 19 der Operationsleuchte 1 angeordnet. Die Mittelachse 19 der Operationsleuchte 1 bildet dabei im Betrieb jene Mittelachse 19 eines in Fig. 2 der Übersichtlichkeit halber nicht weiter dargestellten, aber in Fig. 1 schematisch erkennbaren Leuchtenaufnahmekörpers 16 der Operationsleuchte 1 aus. Der Leuchtenaufnahmekörper 16 ist jener Körper, an dem die Vielzahl an einzelnen Einzelleuchten 3 (umfassend erste Einzelleuchten 3a und zweite Einzelleuchten 3b) aufgenommen / befestigt ist. Folglich sind die Einzelleuchten 3 allesamt an diesem Leuchtenaufnahmekörper 16 befestigt. Im Weiteren ist die Mittelachse 19 auch jene Achse, die sich im Zentrum des Leuchtenaufnahmekörpers 16 der Operationsleuchte 1 befindet und im Wesentlichen entlang einer Handgriffvorrichtung 17 (Fig. 1) der Operationsleuchte 1 erstreckt. Insbesondere ist die Mittelachse 19 dabei jene Achse, entlang welcher sich ein zapfenförmiger Griffabschnitt 21 der nachfolgend näher beschriebenen Handgriffvorrichtung 17, der von dem Operateur berührbar ist, erstreckt.

Somit sind die ersten Einzelleuchten 3a der ersten Leuchtengruppe 11a in einer Umfangsrichtung in Bezug auf die Mittelachse 19 nebeneinander (kettenartig) angeordnet / aneinandergereiht. Alle ersten Einzelleuchten 3a sind dabei derart in einem Winkel relativ zu der Mittelachse 19 ausgerichtet, dass sich deren Längsachsen 6 (die Längsachsen 6 sind jene Achsen, entlang der sich das Lichtstrahlbündel 4 erstreckt) allesamt in einem gemeinsamen Schnittpunkt, hier als erster Schnittpunkt 22 bezeichnet, der in einer gemeinsamen ersten Fokusebene 12 befindlich ist, schneiden. Die Längsachsen 6 der ersten Einzelleuchten 3a weisen daher allesamt einen gleichen Winkel gegenüber der Mittelachse 19 auf. Von dem Leuchtenmodul der Einzelleuchten 3a aus erstreckt sich das jeweilige Lichtstrahlbündel 4 deshalb stets zu der Mittelachse 19 hin, so dass sich der gemeinsamer Fokuspunkt / Schnittpunkt - erster Schnittpunkt 22 - in der ersten Fokusebene 12 ausbildet. Da sich alle ersten Einzelleuchten 3a der ersten Leuchtengruppe 11a in ihrem eingeschalteten Zustand in diesem gemeinsamen, in der ersten Fokusebene 12 befindlichen ersten Schnittpunkt 22 schneiden / überlappen / überdecken, bilden diese ein gemeinsames, erstes rundes Fokuslichtfeld 23 in dieser ersten Fokusebene 12 aus. Die einzelnen ersten Lichtfeldbereiche 8a, die durch die ersten Einzelleuchten 3a jeweils erzeugt werden, überdecken sich somit in dieser ersten Fokusebene 12 vollständig, unter Ausbildung des einen kreisrunden / gemeinsamen ersten Fokuslichtfeldes 23. Das erste Fokuslichtfeld 23 weißt dabei einen maximalen, ersten Durchmesser von ca. 300 mm auf.

Schematisch sind in Fig. 2 zwei sich in Bezug auf die Mittelachse 19 gegenüberliegende erste Einzelleuchten 3a eingeschaltet, so dass eine der ersten Einzelleuchten 3a ein sich entlang einer ersten Längsachse 6a erstreckendes erstes Lichtstrahlbündel 4a erzeugt und eine, um etwa 180° entlang der ersten Umfangslinie 10 versetzt angeordnete, andere erste Einzelleuchten 3a ein sich entlang einer zweiten Längsachse 6b erstreckendes zweites Lichtstrahlbündel 4b ausbildet. Bis zum ersten Schnittpunkt 22 erstrecken sich die beiden Lichtstrahlbündel 4a, 4b aufeinander zu und von einer der Operationsleuchte 1 abgewandten Seite der ersten Fokusebene 12 aus erstrecken sich diese beiden Lichtstrahlbündel 4a und 4b dann wiederum voneinander weg, stets in einem gleichen Winkel (in Bezug auf den Winkelbetrag) zwischen der jeweiligen Längsachse 6a, 6b zur Mittelachse 19 betrachtet.

In einer beabstandet zur ersten Fokusebene 12 angeordneten Ausleuchtebene, hier der Beleuchtungsebene 7, bilden / leuchten die Lichtstrahlbündel 4a und 4b der beiden ersten Einzelleuchten 3a jeweils einen eigenen Lichtfeldbereich 8 aus.

Wie weiterhin in Zusammenwirkung mit Fig. 2 zu erkennen ist, ist neben der ersten Gruppe / Leuchtengruppe 11a der ersten Einzelleuchten 3a eine zweite Leuchtengruppe 11b/Linsengruppe vorgesehen, die wiederum mehrere einzelne Leuchten, d.h. mehrere Einzelleuchten 3 aufweist, wobei diese der zweiten Leuchtengruppe 11b zugehörigen Einzelleuchten als zweite Einzelleuchten 3b nachfolgend bezeichnet sind. Die zweiten Einzelleuchten 3b der zweiten Leuchtengruppe 11b sind wie die ersten Einzelleuchten 3a aufgebaut und funktionierend. Somit weisen auch die zweiten Einzelleuchten 3b jeweils ein Leuchtenmodul mit nur einer LED und einer dieser LED zugeordneten Linse / Linsenoptik auf.

Die zweiten Einzelleuchten 3b der zweiten Leuchtengruppe 11b sind in Bezug auf die Mittelachse 19 gesehen radial außerhalb der ersten Einzelleuchten 3a der ersten Leuchtengruppe 11a angeordnet. Auch die zweiten Einzelleuchten 3b sind auf einer Umfangslinie, die nachfolgend als zweite Umfangslinie 24 bezeichnet ist, kreisförmig nebeneinander angeordnet. Somit sind auch die zweiten Einzelleuchten 3b in Umfangsrichtung der Mittelachse 19 angeordnet. Die zweite Umfangslinie 24 weist folglich einen größeren Durchmesser auf als die erste Umfangslinie 18 auf.

Auch sind wiederum in der zweiten Leuchtengruppe 11b nicht nur zwei (zweite) Einzelleuchten 3b, sondern mehr als zwei (zweite) Einzelleuchten 2b eingesetzt. Insgesamt sind achtzehn zweite Einzelleuchten 2b in der zweiten Leuchtengruppe 11b enthalten und kettenartig entlang der kreisrunden zweiten Umfangslinie 14 aneinander gereiht. Jedoch ist in weiteren Ausführungen auch eine andere Anzahl an zweiten Einzelleuchten 3b in der zweiten Leuchtengruppe 11b umgesetzt, wie mehr als achtzehn oder weniger als achtzehn. Zudem sei darauf verwiesen, dass jede der Einzelleuchten 3; 3a und 3b nicht zwingend eine sich entlang einer kreisförmigen Umfangslinie 18, 24 erstreckende kreisförmige Anordnung aufweisen muss. Auch ist es denkbar, die Einzelleuchten 3a, 3b der Leuchtengruppen 11a, 11b auf andere Weise zueinander anzuordnen, ohne sich von dem Erfindungsgedanken zu entfernen.

Die zweiten Einzelleuchten 3b sind vom Leuchtenaufnahmekörper 16 wiederum allesamt in Richtung der Mittelachse 19 ausgerichtet. Alle zweiten Einzelleuchten 3b schließen wiederum mit ihren Längsachsen 6 der Lichtstrahlbündel 4 einen Winkel mit der Mittelachse 19 ein. Die Längsachsen 6 aller zweiten Einzelleuchten 3b weisen hierbei einen gleichen Winkel gegenüber der Mittelachse 19 auf.

Schematisch sind in Fig. 2 auch zwei zweite Einzelleuchten 3b eingeschaltet, die sich in Bezug auf die zweite Umfangslinie 24 im Wesentlichen um 180° gegenüber liegen. Eine der beiden zweiten Einzelleuchten 3b erzeugt ein als drittes Lichtstrahlbündel 4c bezeichnetes Lichtstrahlbündel 4, das sich entlang der dritten Längsachse 6c erstreckt. Die andere der beiden zweiten Einzelleuchten 3b erzeugt wiederum ein viertes Lichtstrahlbündel 4d, das sich entlang einer vierten Längsachse 6d erstreckt. Die beiden zweiten Einzelleuchten 3b sind derart aufeinander abgestimmt, dass sich ihre Längsachsen 6c und 6d in einem gemeinsamen Fokuspunkt / Schnittpunkt - nachfolgend als zweiter Schnittpunkt 25 bezeichnet - schneiden. Dieser zweite Schnittpunkt 25 liegt in einer zweiten Fokusebene 13, die gegenüber / relativ zu der ersten Fokusebene 12 beabstandet angeordnet ist. Weiterhin schneiden sich nicht nur die Längsachsen 6c, 6d der beiden in Fig. 2 eingeschalteten zweiten Einzelleuchten 3b, sondern alle in der zweite Leuchtengruppe 11b enthaltenen zweiten Einzelleuchten 2b mit ihren Längsachsen 6 in diesem gemeinsamen zweiten Schnittpunkt 25 in der zweiten Fokusebene 13. Da sich alle zweiten Einzelleuchten 3b der zweiten Leuchtengruppe 11b in ihrem eingeschalteten Zustand in diesem gemeinsamen zweiten Schnittpunkt 25 schneiden / überlappen / überdecken, bilden diese ein gemeinsames, zweites rundes Fokuslichtfeld 26 in dieser zweiten Fokusebene 13 aus. Die einzelnen zweiten Lichtfeldbereiche 8b, die durch die zweiten Einzelleuchten 3b jeweils erzeugt werden, überdecken sich somit in dieser zweiten Fokusebene 13 vollständig, unter Ausbildung des einen kreisrunden / gemeinsamen zweiten Fokuslichtfeldes 26. Das zweite Fokuslichtfeld 26 weißt dabei einen maximalen, zweiten Durchmesser von ca. 150 mm auf. In der beabstandet zur zweiten Fokusebene 12 angeordneten Beleuchtungsebene 7, bilden / leuchten die Lichtstrahlbündel 4c und 4d der beiden zweiten Einzelleuchten 3b jeweils einen eigenen Lichtfeldbereich 8b aus.

Jede Fokusebene 12 bzw. 13 ist in dieser Ausführung als eine Normalebene relativ zur Mittelachse 11 ausgestaltet, d.h., dass die Mittelachse 19 senkrecht zu beiden (parallel zueinander angeordneten) Fokusebenen 12, 13 ausgerichtet ist. Die erste Fokusebene 12 ist, entlang der Mittelachse 19 betrachtet, näher an der Operationsleuchte 1, d.h. an dem Leuchtenaufnahmekörper 16 der Operationsleuchte 1, angeordnet als die zweite Fokusebene 13. Daher weist die erste Fokusebene 12 einen geringeren Abstand entlang der Mittelachse 19 relativ zu der Operationsleuchte 1 auf als die zweite Fokusebene 13. In einer besonders vorteilhaften Ausführungsform ist der Abstand der ersten Fokusebene 12 entlang der Mittelachse 19 zur Operationsleuchte 1 / zum Leuchtenaufnahmekörper 16 1m und der Abstand der zweiten Fokusebene 13 entlang der Mittelachse 19 relativ zur Operationsleuchte 1 / zum Leuchtenaufnahmekörper 16 1,20m, besonders bevorzugt 1,40m.

Die Einzelleuchten 3a, 3b bilden in dieser Ausführung elliptische Lichtfeldbereiche 8a, 8b aus. In einer weiteren Ausführungsform sind die Lichtfeldbereiche 8a, 8b teilweise eckig, etwa rautenförmig ausgebildet.

In einer schematisch dargestellten Beleuchtungsebene 7, die sich (in axialer Richtung der Mittelachse 19 betrachtet) zwischen den beiden Fokusebenen 12, 13 in diesem Ausführungsbeispiel befindet, bilden aufgrund des Abstandes der Beleuchtungsebene 7 zu den Fokusebenen 12, 13 die Einzelleuchten 3a, 3b beider Leuchtengruppen 11a, 11b allesamt jeweils einen Lichtfeldbereich 8 aus. Die beiden in Fig. 2 eingeschalteten ersten Einzelleuchten 3a erzeugen in der Beleuchtungsebene 7 je den ersten Lichtfeldbereich 8a, während die eingeschalteten zweiten Einzelleuchten 3b wiederum je einen zweiten Lichtfeldbereich 8b erzeugen. Die Lichtfeldbereiche 8a und 8b überlappen, d.h. überdecken sich teilweise gegenseitig. Z.B. überdeckt der zweite Lichtfeldbereich 8b einer der zweiten Einzelleuchten 3b alle anderen Lichtfeldbereiche 8a, 8b der weiteren ersten und zweiten Einzelleuchten 3a, 3b.

Somit ist ein Gesamtlichtfeld 5 der in Fig. 2 angesteuerten Einzelleuchten 3a, 3b durch Kombination der einzelnen Lichtfeldbereiches 8a, 8b erzeugt. In Abhängigkeit der bestromten ersten und/oder zweiten Einzelleuchten 3a, 3b ist das Gesamtlichtfeld 5 somit in verschiedenen Lichtfeldanordnungen / Lichtfeldgeometrien ausbildbar, wobei die Lichtfeldgeometrie 27 (d.h. die Geometrie des Gesamtlichtfeldes 5) in diesem Beispiel linienförmig ausgebildet ist, wobei die einzelnen Lichtfeldbereiche 8a, 8b der Einzelleuchten 3a, 3b linienförmig (unter gegenseitiger, teilweiser Überdeckung) aneinander gereiht sind.

Die Einzelleuchten 3a und 3b der ersten Leuchtengruppe 11a und der zweiten Leuchtengruppe 11b, sowie alle weiteren Einzelleuchten 3, falls vorhanden, sind innerhalb der Leuchtengruppe 11a, 11b als auch zwischen den Leuchtengruppen 11a, 11b unabhängig voneinander bestrombar, d.h. elektrisch ansteuerbar / betätigbar, sodass sich das erzeugte Gesamtlichtfeld 5 in der Beleuchtungsebene 7 beliebig geometrisch einstellen lässt. Unter einer Einstellung der Lichtfeldgeometrie 27 ist hierbei sowohl eine Veränderung der Form bzw. Proportionen des Gesamtlichtfeldes 5 (d.h. der einzelnen Lichtfeldbereiche 8; 8a, 8b) als auch eine Veränderung der Ausrichtung des Gesamtlichtfeldes 5 (d.h. der Lichtfeldgeometrie 18) innerhalb der Beleuchtungsebene 7 zu verstehen.

Zur Ansteuerung der Einzelleuchten 3; 3a, 3b ist weiterhin eine Steuereinheit 10 in der Operationsleuchte 1, nämlich in dem Leuchtenaufnahmekörper 16 der Operationsleuchte 1, integriert / enthalten. Der innere Aufbau der Operationsleuchte 1 sowie des Leuchtenaufnahmekörpers 16 ist besonders gut in Fig. 1 zu erkennen. In Fig. 1 sind die einzelnen Einzelleuchten 3a, 3b nicht mehr einzeln dargestellt, sondern vereinfacht durch abgebildete Blöcke schematisch angedeutet. Auch die elektrische Verbindung zwischen den Einzelleuchten 3a, 3b und der Steuereinheit 10 ist hierbei lediglich schematisch mittels zweier elektrischer Leitungen 28 angedeutet.

In dieser Ausführung ist, wobei dies der Übersichtlichkeit halber nicht weiter dargestellt ist, die Steuereinheit 10 mit jeder Leuchtengruppe 11a, 11b und insbesondere mit jeder einzelnen Einzelleuchte 3a, 3b der Leuchtengruppen 11a, 11b elektrisch sowie datenübermittelnd verbunden. Dadurch ist gewährleistet, dass die Steuereinheit 10 die Einzelleuchten 3a, 3b in Abhängigkeit eines generierten Steuerbefehls je nach Bedarf einzeln bestromt bzw. ein- und ausschaltet. Somit sind die Einzelleuchten 3a, 3b untereinander unabhängig voneinander bestrombar. Die Steuereinheit 10 ist weiterhin mit einer Helligkeitserfassungseinrichtung 9 verbunden. Die Steuereinheit 10 ist mittels einer kabellosen Datenübertragung / Datenverbindung 29 in Form einer Funkverbindung mit der Helligkeitserfassungseinrichtung 9 verbunden.

Die Helligkeitserfassungseinrichtung 9 ist jene Einrichtung, die ausgebildet ist, die Helligkeit in dem Gesamtlichtfeld 5 zu messen / zu ermitteln. Die Steuereinheit 10 ist im Betrieb der Operationsleuchte 1 dauerhaft über die Datenübertragung 29 datenübermittelnd mit der Helligkeitserfassungseinrichtung 9 verbunden. Die durch die Helligkeitserfassungseinrichtung 9 in einem Messbereich 14 des Gesamtlichtfeldes 5 ermittelten / gemessenen Helligkeitswerte, die nachfolgend als Ist-Helligkeitswerte bezeichnet sind, werden anschließend über die Datenübertragung 29 an die Steuereinheit 10 übertragen. Die Steuereinheit 10 ist dabei derart ausgebildet, dass sie - in Abhängigkeit des zumindest einen durch die Helligkeitserfassungseinrichtung 9 ermittelten Ist-Helligkeitswertes - steuernd, nämlich beleuchtungsstärkesteuernd, auf die jeweiligen Einzelleuchten 3a, 3b, einwirkt. Die Steuereinheit 10 wirkt dabei auf die Einzelleuchten 3a, 3b unabhängig von den übrigen Einzelleuchten 3a, 3b ein. Somit ist die Helligkeitserfassungseinrichtung 9 sowie die Steuereinheit 10 erfindungsgemäß derart ausgebildet und die Steuereinheit 10 erfindungsgemäß derart mit den Einzelleuchten 3; 3a, 3b verbunden (d.h. die Steuereinheit 10 wirkt derart steuernd auf die Einzelleuchten 3 ein), dass abhängig von dem zumindest einen Ist-Helligkeitswert die Beleuchtungsstärke einer ersten Einzelleuchte 3a gezielt und unabhängig von der Beleuchtungsstärke einer der zweiten Einzelleuchten 3b zum Erhellen oder Verdunkeln (d.h. zum Verändern der Helligkeit) des ersten Lichtfeldbereiches 8a einstellbar ist.

Zu diesem Zwecke ist die Helligkeitserfassungseinrichtung 9 derart ausgerichtet, dass ein durch die Helligkeitserfassungseinrichtung 9 ermittelter Messbereich 14 stets innerhalb des Gesamtlichtfeldes 5 angeordnet ist, so dass sichergestellt ist, dass auch stets nur ein Messbereich 14 erfasst wird, der relevant für die Beleuchtung der Beleuchtungsebene 7 ist.

Die Helligkeitserfassungseinrichtung 9 weist zur Messung / zur Aufnahme des Messbereiches 14 eine Kamera 15 auf, die nachfolgend auch als erste Kamera 15 bezeichnet ist. Die Kamera 15 umfasst auf übliche Weise ein Objektiv / eine Optik sowie eines Messsensors in Form eines Helligkeitssensors / Lichtsensors, der hier der Übersichtlichkeit halber nicht weiter dargestellt ist. Der Helligkeitssensor an sich muss jedoch nicht innerhalb einer Kamera 15, wie es hier ausgeführt ist, integriert sein. In weiteren Ausführungen ist der Helligkeitssensor ohne Objektiv ausgestaltet und somit als loser Helligkeitssensor vorhanden und in dem Leuchtenaufnahmekörper 16 integriert. Der Helligkeitssensor weist in dieser Ausführung mehrere Fototransistoren auf, wobei er auch alternativ als ein Fotowiderstand oder eine Fotodiode oder eine Gruppe von Fotowiderständen oder von Fotodioden ausgebildet sein kann.

Wie in Fig. 1 weiterhin besonders gut zu erkennen ist, definiert die Ausrichtung der Kamera 15, insbesondere deren Objektivs die Position des Messbereiches 14. In dieser Ausführung ist die erste Kamera 15 innerhalb der Handgriffvorrichtung 17 angeordnet, d.h. die Kamera 15 ist in der Handgriffvorrichtung 17 integriert. Die Handgriffvorrichtung 17 ist dabei jene Vorrichtung, die im Betrieb der Operationsleuchte 1 fest mit dem Leuchtenaufnahmekörper 16 verbunden ist. Durch die Anordnung der Einzelleuchten 3 in einer festen Ausrichtung an dem Leuchtenaufnahmekörper 16 ist es möglich, durch Anfassen der Handgriffvorrichtung 17 sowie durch Aufbringen einer gewissen Verstellkraft, die Operationsleuchte 1 samt Leuchtenaufnahmekörper 16 im Betrieb / in der Operationsphase einzustellen. Die Handgriffvorrichtung 17 ist samt ihres Griffabschnittes 21, der im Wesentlichen röhrenförmig ausgebildet ist, wieder abnehmbar an dem Leuchtenaufnahmekörper 16 angebracht. Dadurch ist die Handgriffvorrichtung 17 gesamtheitlich leicht sterilisierbar. Die Kamera 15 ist dabei innerhalb des röhrenförmigen / hohl ausgebildeten Griffabschnittes 21 eingesetzt / aufgenommen. Der Griffabschnitt 21 erstreckt sich im Wesentlichen entlang der Mittelachse 19, d.h. senkrecht zu einer gedachten Befestigungsebene 20 an dem Leuchtenaufnahmekörper 16. An einer, dem Leuchtenaufnahmekörper 16 abgewandten Seite, ist der Griffabschnitt 21 ausgespart, d.h. er weist eine Öffnung auf, sodass die Kamera 15 mit ihrem Objektiv den Messbereich 14 auf der Beleuchtungsebene 7 erfassen kann. Die Kamera 15 ist somit ebenfalls entlang dieser Mittelachse 19 ausgerichtet.

Die Kamera 15 ist weiterhin derart ausgebildet, dass sie ein festes Objektiv / eine feste Optik aufweist, das / die eine feste / fest eingestellte Brennweite aufweist. Die erste Kamera 15 ist hinsichtlich ihrer Belichtungszeit einstellbar, so dass sie in Abhängigkeit der Gesamthelligkeit des Messbereiches 14 mehrere Ist-Helligkeitswerte stets verlässlich misst. Wie hierbei mittels der Rückstrahlen 30 schematisch dargestellt ist, wird ein gewisser Anteil des ursprünglich (durch die Lichtstrahlbündel 4) von den Einzelleuchten 3a, 3b imitierten Lichtes reflektiert und von der Kamera 15 gemessen / ermittelt / erfasst / aufgenommen.

Weiterhin weist die Kamera einen festen / fest eingestellten Blendenwert auf, so dass entsprechend der Belichtungszeitwert automatisch nachgeregelt wird, um stets den fest eingestellte Blendenwert zu erreichen. Alternativ, in einer weiteren Ausführung ist die Kamera 15 auch derart ausgebildet, dass sie statt des fest eingestellten Blendenwerts einen einstellbaren Blendenwert aufweist und stattdessen die Belichtungszeit fest ist, d.h. fest eingestellt ist. Dadurch wird dann die Autoirisfunktion der Kamera 15 zur Belichtungsmessung genutzt. Dadurch ist stets immer nur ein Wert der Kamera 15 einzustellen, um ein verlässliches Bild des Messbereiches 14 zu erzeugen.

Anhand des durch die Kamera 15 aufgenommenen Bildes des Messbereiches 14 werden durch Auswertung der einzelnen, erfassten Pixel-Helligkeiten durch die Steuereinheit 10 mehrere, jeweils zumindest einem Teilbereich des Messbereiches 14 zugeordnete Ist-Helligkeitswerte erfasst / ermittelt, die zur Gesamthelligkeit des Messbereiches 14 beitragen. Je nach Helligkeit der einzelnen Teilbereiches des Messbereiches 14 wird dann die Beleuchtungsstärke der den jeweiligen Teilbereich mit seinem Lichtfeldbereich 8a, 8b ausleuchtenden Einzelleuchte 3a, 3b nachgeregelt / angepasst.

Somit ist die Kamera 15 derart ausgestaltet, dass sie in dieser Ausführung den Messbereich 14 gleichzeitig erfasst und dem in mehrere Teilbereiche / Teilflächen unterteilten Messbereich 14 / Bildbereich / Bild mehrere Ist-Helligkeitswerte (je Teilbereich einen Ist-Helligkeitswert) zuordnet. Der Messbereich 14 der Kamera 15 ist folglich in mehrere Punktbereiche, d.h. Spots, eingeteilt, wonach dann die verschiedenen Ist-Helligkeitswerte der unterschiedlichen Teilbereiche des Messbereiches 14 ermittelt werden. Alternativ hierzu ist es jedoch auch möglich, dass der Messbereich 14 integral erfasst wird und ein einziger Ist-Helligkeitswert aus diesem Messbereichs 14 ermittelt wird. Dann sind vorzugsweise mehrere Kameras 15 vorhanden, die jeweils einen Messbereich 14 aufnehmen und einen Ist-Helligkeitswert an die Steuereinheit 10 übertragen.

Innerhalb der Steuereinheit 10 sind wiederum verschiedene Logarithmen hinterlegt, nach denen, in Abhängigkeit der ermittelten Ist-Helligkeitswerte, die Steuereinheit 10 beleuchtungsstärkesteuernd auf die Einzelleuchten 3a, 3b, die mit ihren Lichtstrahlbündeln 4 das Gesamtlichtfeld 5 mit ausbilden, zur Einstellung ihrer Beleuchtungsstärke, einwirkt. Detektiert die Kamera 15 bspw. einen zu hellen Ist-Helligkeitswert in einem Teilbereich des Messbereiches 14, wird die Einzelleuchte 3a, 3b oder die mehreren Einzelleuchten 3a, 3b, die den Teilbereich des Messbereiches 14 mit beleuchten, entsprechend abgedunkelt, d.h. in ihrer Beleuchtungsstärke reduziert. Im Gegensatz dazu ist die Steuereinheit 10 bei einem zu dunklen Helligkeitswert derart wirkend, dass der jeweilige Teilbereich des Messbereiches 14 dann wiederum die Einzelleuchten 3 unabhängig voneinander erhellt, d.h. in ihrer Beleuchtungsstärke erhöht.

In dieser Ausführungsform weist die Helligkeitserfassungseinrichtung 9 nicht nur die erste Kamera 15, sondern auch eine zweite Kamera 31 auf, die jedoch wiederum wie die erste Kamera 15 aufgebaut sowie funktionierend ist. Auch ist diese zweite Kamera 31 wiederum mittels einer Datenübertragung 29 mit der Steuereinheit 10 verbunden. Die zweite Kamera 31 ist jedoch nicht innerhalb der abnehmbaren Handgriffvorrichtung 17 integriert, sondern an dem / in dem Leuchtenaufnahmekörper 16 angebracht. In dieser Ausführung ist die zweite Kamera 31 an einer in Bezug auf die Mittelachse 19 betrachteten radialen Außenseite des Leuchtenaufnahmekörpers 16 positioniert. Die zweite Kamera 31 ist auch nicht mit ihrem Sichtfeld parallel bzw. so wie die erste Kamera 15 koaxial zu der Mittelachse 19 ausgerichtet, sondern mit ihrem Objektiv schräg zu der Mittelachse 19 ausgerichtet. Dadurch nimmt die zweite Kamera 32 einen etwas größeren (zweiten) Messbereich 32 als die erste Kamera 15 auf. Der Messbereich der zweiten Kamera ist nachfolgend als zweiter Messbereich 32 bezeichnet. Die beiden Kameras 15 und 31, sind hierbei derart ausgerichtet, dass sich ihre Messbereiche 14 versetzt zueinander angeordnet sind. D.h., die beiden Messbereiche 14 und 32 sind nicht deckungsgleich, sondern im Wesentlichen versetzt zueinander ausgerichtet. Dies ermöglicht eine Aufnahme mittels der zweiten Kamera 31 eines weiteren, unabhängig von dem ersten Messbereich 14, jedoch wiederum innerhalb des Gesamtlichtfeldes 5 angeordneten Messbereich 32 zu ermitteln. Auch die zweite Kamera 31 detektiert wiederum die Rückstrahlung 30.

Der Vollständigkeit halber sei zudem erwähnt, dass die beiden Kameras 15, 31 oder auch zumindest die erste Kamera 15 oder die zweite Kamera 31 einzeln als Leuchtdichtemessgeräte ausgebildet sein können, wodurch die Helligkeitserfassungseinrichtung 9 noch effektiver arbeitet.

Im Weiteren erfasst die Helligkeitserfassungseinrichtung 9, nämlich die jeweilige Kameras 15, 31 die Ist-Helligkeitswerte kontinuierlich / dauerhaft, d.h. mit mehreren Bildern pro Sekunde. Alternativ hierzu ist es auch möglich, dass entweder die erste Kamera 15 oder die zweite Kamera 31 oder beide Kameras 15 und 31 deren Messbereiche 14 und 32 intervallartig, bspw. in Aufnahmezeitabstände von mehreren Sekunden oder Minuten zwischen den Einzelaufnahmen oder weiter alternativ durch manuelle Eingabe eines Aufnahmebefehls an einer Betätigungsvorrichtung erfassen. Dadurch ist es möglich, dass die Kameras 15, 31 noch effizienter arbeiten.

In anderen Worten ausgedrückt, ist es somit möglich, die Blendenfunktion einer Kamera 15, 31 auszuwerten und damit eine Regelungsfunktion zu realisieren. Weiterhin sind Sensoren und Messgeräte verwendbar, mit denen auch die Leuchtdichte bzw. die Helligkeit einer Oberfläche der Beleuchtungsebene 7 gemessen werden kann. Die Lichtintensität wird erfindungsgemäß automatisch dem Operationsbereich / OP-Feld, d.h. der Beleuchtungsebene 7 angepasst. Die automatische Lichtintensität / Helligkeitsregelung wirkt sich schonend auf das Operationsfeld aus, da keine unnötige Bestrahlung ins Operationsfeld gelangt und durch die Erwärmung etwa gar ein Austrocknungseffekt entstehen kann. Auch ist ein augenschonendes und ermüdungsfreies Arbeiten des Operateurs dadurch umgesetzt, da im Wesentlichen keine Blendungen entstehend und unnötige hohe Beleuchtungsstärken vermieden werden.

Die Auswertung der Bildinformation der Kamera 15, 31 oder eines geeigneten Sensors wird hierbei durchgeführt. Anhand einer Autoirisfunktion (Belichtungsmessung) der Kamera 15, 31, die auf das Operationsfeld gerichtet ist, kann somit die Lichtintensität nachgeregelt werden und durch die entsprechende Leuchtenelektronik sowie die Operationsleuchte 1 auf einen entsprechenden Wert eingestellt werden. Eine berührungslose Messung der Helligkeit bzw. der reflektierenden Strahlung im Operationsfeld ist dadurch möglich. Die Helligkeit kann mittels Leuchtdichtemessgerät bzw.

Lichtsensor / Fotodiode / Fototransistor / Fotowiderstand ausgeführt werden, welcher die reflektierte Lichtstrahlung der beleuchteten Objektoberfläche misst. Ein Nachregeln der Lichtintensität / Beleuchtungsstärke der Operationsleuchte 1 findet dann auf einem entsprechenden Wert statt. Die Justierung dieses Systems kann auch durch eine manuelle Einstellung der maximalen Beleuchtungsstärke erfolgen, bei der z.B. auf einem weißen Untergrund noch keine Blendung entsteht. Bei einer Umschaltung auf Automatik wird dann auf diese Beleuchtungsstärke referenziert und eine Blendung auch auf anderen Untergründen vermieden.

Ist die Operationsleuchte 1 mit einer medizinischen Kamera 15 ,31 zur Bildgebung ausgestattet, können die Blendenwerte automatisch ausgelesen werden und zur Regelung der Helligkeit direkt herangezogen werden. Der Blendenwert kann dabei als Spot oder Integral ermittelt werden. Denkbar ist es auch eine Bilderkennung der hellsten Stelle (die Blendung) zu ermitteln und diese dann für eine Korrektur zu bewerten. Ist die Operationsleuchte 1 hingegen nicht mit einer Kamera 15, 31 für die Bildgebung ausgestattet, kann diese Funktion ein Sensor oder eine sehr günstige Kamera übernehmen. Denkbar ist dabei auch, dass diese Funktion die Dimmung kontinuierlich beeinflusst. Dies könnte jedoch z.B. bei der Verwendung heller Operationshandschuhe und sich damit ständig ändernder Lichtverhältnisse zu Problemen führen. Denkbar wäre daher, dies zu vermeiden, wobei dann ein Abgleich in entsprechenden Zeitabständen / Zeitintervallen umgesetzt ist. Denkbar wäre auch eine manuelle Auslösung der Messung, z.B. am zentralen sterilisierbaren Handgriff / der Handgriffvorrichtung 17. Hierzu wird idealerweise der Blick auf das Operationsfeld freigegeben, um dann die Messung auszulösen. Die Leuchte 1 stellt dann ihre Helligkeit automatisch auf die aktuelle Situation ein.

### Bezugszeichen

- 1: Operationsleuchte
- 2: Operationsfeld
- 3: Einzelleuchte
- 3a: erste Einzelleuchte
- 3b: zweite Einzelleuchte
- 4: Lichtstrahlbündel
- 4a: erstes Lichtstrahlbündel
- 4b: zweites Lichtstrahlbündel
- 4c: drittes Lichtstrahlbündel
- 4d: viertes Lichtstrahlbündel
- 5: Gesamtlichtfeld
- 6: Längsachse
- 6a: erste Längsachse
- 6b: zweite Längsachse
- 6c: dritte Längsachse
- 6d: vierte Längsachse
- 7: Beleuchtungsebene
- 8: Lichtfeldbereich
- 8a: erster Lichtfeldbereich
- 8b: zweiter Lichtfeldbereich
- 9: Helligkeitserfassungseinrichtung
- 10: Steuereinheit
- 11a: erste Leuchtengruppe
- 11b: zweite Leuchtengruppe
- 12: erste Fokusebene
- 13: zweite Fokusebene
- 14: Messbereich
- 15: Kamera / erste Kamera
- 16: Leuchtenaufnahmekörper
- 17: Handgriffvorrichtung
- 18: erste Umfangslinie
- 19: Mittelachse
- 20: Befestigungsebene
- 21: Griffabschnitt
- 22: erster Schnittpunkt
- 23: erstes Fokuslichtfeld
- 24: zweite Umfangslinie
- 25: zweiter Schnittpunkt
- 26: zweites Fokuslichtfeld
- 27: Lichtfeldgeometrie
- 28: Leitung
- 29: Datenübertragung
- 30: Rückstrahl / Rückstrahlung
- 31: zweite Kamera
- 32: zweiter Messbereich

## Patentansprüche

1. Operationsleuchte (1) zum Ausleuchten eines Operationsfeldes (2), mit
- einer Vielzahl an Einzelleuchten (3), die in einem eingeschalteten Zustand jeweils ein sich entlang einer Längsachse (6) erstreckendes sowie in einer Beleuchtungsebene (7) einen Lichtfeldbereich (8) erzeugendes Lichtstrahlbündel (4) ausbilden, wobei die Lichtfeldbereiche (8) der Einzelleuchten (3) in der Beleuchtungsebene (7) so nebeneinander und/oder zumindest teilweise übereinander angeordnet sind, dass ein Gesamtlichtfeld (5) gebildet ist,
- einer Helligkeitserfassungseinrichtung (9), die ausgebildet ist, einen Ist-Helligkeitswert in dem Gesamtlichtfeld (5) zu ermitteln, sowie
- einer Steuereinheit (10), die in Abhängigkeit des ermittelten Ist-Helligkeits wertes beleuchtungsstärkesteuernd auf die Einzelleuchten (3) einwirkt,
wobei die Helligkeitserfassungseinrichtung (9) sowie die Steuereinheit (10) derart ausgebildet sind und die Steuereinheit (10) derart mit den Einzelleuchten (3) verbunden ist, dass abhängig von dem Ist-Helligkeitswert eine Beleuchtungsstärke einer ersten Einzelleuchte (3a) gezielt und unabhängig von einer Beleuchtungsstärke einer zweiten Einzelleuchte (3b) zum Erhellen oder Verdunkeln eines ersten Lichtfeldbereiches (8a) einstellbar ist, **dadurch gekennzeichnet, dass** die Helligkeitserfassungseinrichtung (9) eine Kamera (15) aufweist und die Kamera (15) ein Objektiv mit einer fest eingestellten Brennweite aufweist.

2. Operationsleuchte (1) nach Anspruch 1, **dadurch gekennzeichnet, dass** die Einzelleuchten (3) in mehrere Leuchtengruppen (11a, 11b) aufgeteilt sind, wobei mehrere einer ersten Leuchtengruppe (11a) zugeordnete Einzelleuchten (3a) derart ausgerichtet und zueinander angeordnet sind, dass sich die Längsachsen (6) ihrer Lichtstrahlbündel (4) in einer ersten gemeinsamen Fokusebene (12) schneiden.

3. Operationsleuchte (1) nach Anspruch 2, **dadurch gekennzeichnet, dass** mehrere einer zweiten Leuchtengruppe (11b) zugeordnete Einzelleuchten (3b) derart ausgerichtet und zueinander angeordnet sind, dass sich die Längsachsen (6) ihrer Lichtstrahlbündel (4) in einer, zu der ersten Fokusebene (12) beabstandet angeordneten, zweiten gemeinsamen Fokusebene (13) schneiden.

4. Operationsleuchte (1) nach Anspruch 2 und 3, **dadurch gekennzeichnet, dass** die Steuereinheit (10) elektrisch mit den Einzelleuchten (3a) der ersten Leuchtengruppe (11a) und/oder den Einzelleuchten (3b) der zweiten Leuchtengruppe (11b) verbunden ist.

5. Operationsleuchte (1) nach einem der Ansprüche 1 bis 4, **dadurch gekennzeichnet, dass** die Helligkeitserfassungseinrichtung (9) zumindest einen Helligkeitssensor umfasst, der zumindest einen Fototransistor, zumindest einen Fotowiderstand und/oder zumindest eine Fotodiode aufweist.

6. Operationsleuchte (1) nach einem der Ansprüche 1 bis 5, **dadurch gekennzeichnet, dass** die Helligkeitserfassungseinrichtung (9) zumindest einen Messbereich (14) innerhalb des Gesamtlichtfeldes (5) erfasst, welcher Messbereich (14) eine kleinere Fläche aufweist als das Gesamtlichtfeld (5).

7. Operationsleuchte (1) nach Anspruch 6, **dadurch gekennzeichnet, dass** die Helligkeitserfassungseinrichtung (9) den Ist-Helligkeitswert des Messbereiches (14) gesamtheitlich oder ausschnittsweise ermittelt.

8. Operationsleuchte (1) nach einem der Ansprüche 1 bis 7, **dadurch gekennzeichnet, dass** ein Blendenwert der Kamera (15) fest eingestellt ist oder einstellbar ist.

9. Operationsleuchte (1) nach einem der Ansprüche 1 bis 8, **dadurch gekennzeichnet, dass** ein Belichtungszeitwert der Kamera (15) fest eingestellt ist oder einstellbar ist.

10. Operationsleuchte (1) nach einem der Ansprüche 1 bis 9, **dadurch gekennzeichnet, dass** die Helligkeitserfassungseinrichtung (9) ein Leuchtdichtemessgerät umfasst oder als solches ausgebildet ist.

11. Operationsleuchte (1) nach einem der Ansprüche 1 bis 10, **dadurch gekennzeichnet, dass** die Helligkeitserfassungseinrichtung (9) in einem die Einzelleuchten (3) aufnehmenden Leuchtenaufnahmekörper (16) eingesetzt ist oder in einer mit dem Leuchtenaufnahmekörper (19) wiederabnehmbar verbindbaren Handgriffvorrichtung (17) integriert ist.

12. Operationsleuchte (1) nach einem der Ansprüche 1 bis 11, **dadurch gekennzeichnet, dass** die Helligkeitserfassungseinrichtung (9) den Ist-Helligkeitswert kontinuierlich, intervallartig oder nach manueller Eingabe eines Aufnahmebefehls erfasst.

13. Verfahren zur Steuerung einer Operationsleuchte (1) zum Ausleuchten eines Operationsfeldes (2), wobei die Operationsleuchte (1)
- eine Vielzahl an Einzelleuchten (3), die in einem eingeschalteten Zustand jeweils ein sich entlang einer Längsachse (6) erstreckendes sowie in einer Beleuchtungsebene (7) einen Lichtfeldbereich (8) erzeugendes Lichtstrahlbündel (4) ausbilden, wobei die Lichtfeldbereiche (8) der Einzelleuchten (3) in der Beleuchtungsebene (7) so nebeneinander und/oder zumindest teilweise übereinander angeordnet sind, dass ein Gesamtlichtfeld (5) gebildet ist,
- eine Helligkeitserfassungseinrichtung (9), die ausgebildet ist, einen Ist-Helligkeitswert in dem Gesamtlichtfeld (5) zu ermitteln, wobei die Helligkeitserfassungseinrichtung (9) eine Kamera (15) aufweist und die Kamera (15) ein Objektiv mit einer fest eingestellten Brennweite aufweist sowie
- eine Steuereinheit (10), die in Abhängigkeit des ermittelten Ist-Helligkeitswertes beleuchtungsstärkesteuernd auf die Einzelleuchten (3) einwirkt, aufweist,
wobei die Helligkeitserfassungseinrichtung (9) sowie die Steuereinheit (10) derart ausgebildet sind und die Steuereinheit (10) derart mit den Einzelleuchten (3) verbunden ist, dass abhängig von dem Ist-Helligkeitswert eine Beleuchtungsstärke einer ersten Einzelleuchte (3a) gezielt und unabhängig von einer Beleuchtungsstärke einer zweiten Einzelleuchte (3b) zum Erhellen oder Verdunkeln eines ersten Lichtfeldbereiches (8a) eingestellt wird.

## Claims

1. A surgical lamp (1) for illuminating an operating area (2), comprising
- a plurality of individual lamps (3) which, in an activated state, form a respective light beam bundle (4) extending along a longitudinal axis (6) and generating a light field region (8) in an illumination plane (7), wherein the light field regions (8) of the individual lamps (3) are arranged next to one another and/or at least partially over one another in the illumination plane (7) in such way that a total light field (5) is formed,
- a brightness detection device (9) which is designed to determine an actual brightness value in the total light field (5), as well as
- a control unit (10) which acts on the individual lamps (3) such that the illumination intensity is controlled in accordance with the determined actual brightness value,
wherein the brightness detection device (9) and the control unit (10) are designed in such way and the control unit (10) is connected to the individual lamps (3) in such way that an illumination intensity of a first individual lamp (3a) is adjustable in a targeted manner depending on the actual brightness value and is adjustable for brightening or dimming a first light field region (8a) independently of an illumination intensity of a second individual lamp (3b),
**characterized in that** the brightness detection device (9) includes a camera (15), wherein the camera (15) includes a lens having a fixedly set focal length.

2. The surgical lamp (1) according to claim 1, **characterized in that** the individual lamps (3) are divided into plural lamp groups (11a, 11b), wherein plural individual lamps (3a) associated with a first lamp group (11a) are oriented and arranged relative to one another so that the longitudinal axes (6) of the light beam bundles (4) thereof intersect in a first common focal plane (12).

3. The surgical lamp (1) according to claim 2, **characterized in that** plural individual lamps (3b) associated with a second lamp group (11b) are oriented and arranged relative to each other so that the longitudinal axes (6) of the light beam bundles (4) thereof intersect in a second common focal plane (13) arranged at a distance from the first focal plane (12).

4. The surgical lamp (1) according to claim 2 and 3, **characterized in that** the control unit (10) is electrically connected to the individual lamps (3a) of the first lamp group (11a) and/or to the individual lamps (3b) of the second lamp group (11b).

5. The surgical lamp (1) according to any one of the claims 1 to 4, **characterized in that** the brightness detection device (9) comprises at least one brightness sensor including at least one phototransistor, at least one photoresistor and/or at least one photodiode.

6. The surgical lamp (1) according to any one of the claims 1 to 5, **characterized in that** the brightness detection device (9) detects at least one measuring area (14) within the total light field (5), which measuring area (14) has a smaller surface area than the total light field (5).

7. The surgical lamp (1) according to claim 6, **characterized in that** the brightness detection device (9) determines the actual brightness value of the measuring area (14) in total or in part.

8. The surgical lamp (1) according to any one of the claims 1 to 7, **characterized in that** a f-number of the camera (15) is fixedly set or settable.

9. The surgical lamp (1) according to any one of the claims 1 to 8, **characterized in that** an exposure time value of the camera (15) is fixedly set or settable.

10. The surgical lamp (1) according to any one of the claims 1 to 9, **characterized in that** the brightness detection device (9) comprises a luminance meter or is in the form of the latter.

11. The surgical lamp (1) according to any one of the claims 1 to 10, **characterized in that** the brightness detection device (9) is inserted in a lamp receiving member (16) receiving the individual lamps (3) or is integrated in a handle device (17) adapted to be detachably connected to the lamp receiving member (19).

12. The surgical lamp (1) according to any one of the claims 1 to 11, **characterized in that** the brightness detection device (9) detects the actual brightness value continuously, at intervals or upon manual input of a recording command.

13. A method for controlling a surgical lamp (1) for illuminating an operating area (2), with the surgical lamp (1) comprising
- a plurality of individual lamps (3) which, in an activated state, form a respective light beam bundle (4) extending along a longitudinal axis (6) and generating a light field region (8) in an illumination plane (7), wherein the light field regions (8) of the individual lamps (3) are arranged next to one another and/or at least partially over one another in the illumination plane (7) in such way that a total light field (5) is formed,
- a brightness detection device (9) which is designed to determine an actual brightness value in the total light field (5), wherein the brightness detection device (9) includes a camera (15) and the camera (15) includes a lens having a fixedly set focal length, as well as
- a control unit (10) which acts on the individual lamps (3) such that the illumination intensity is controlled in accordance with the determined actual brightness value, wherein the brightness detection device (9) and the control unit (10) are designed in such way and the control unit (10) is connected to the individual lamps (3) in such way that an illumination intensity of a first individual lamp (3a) is set in a targeted manner depending on the actual brightness value and is set for brightening or dimming a first light field region (8a) independently of an illumination intensity of a second individual lamp (3b).

## Revendications

1. Éclairage opératoire (1) destiné à éclairer un champ opératoire (2), comprenant
- une pluralité de luminaires individuels (3), qui, dans un état allumé, forment respectivement un faisceau lumineux (4) s'étendant le long d'un axe longitudinal (6) et produisant une zone de champ lumineux (8) dans un plan d'éclairage (7), dans lequel les zones de champ lumineux (8) des luminaires individuels (3) sont agencées dans le plan d'éclairage (7) les unes à côté des autres et/ou au moins en partie les unes au-dessus des autres de telle sorte qu'un champ lumineux total (5) est formé,
- un dispositif de détection de luminosité (9), qui est conçu pour déterminer une valeur de luminosité réelle dans le champ lumineux total (5), ainsi que
- une unité de commande (10) qui, en fonction de la valeur de luminosité réelle déterminée, agit sur les luminaires individuels (3) par une commande d'intensité d'éclairage,
dans lequel le dispositif de détection de luminosité (9) ainsi que l'unité de commande (10) sont conçus de telle sorte et l'unité de commande (10) est reliée aux luminaires individuels (3) de telle sorte que, en fonction de la valeur de luminosité réelle, une intensité d'éclairage d'un premier luminaire individuel (3a) peut être réglée de façon ciblée et indépendamment d'une intensité d'éclairage d'un deuxième luminaire individuel (3b) pour rendre plus claire ou plus sombre une première zone de champ lumineux (8a), **caractérisé en ce que** le dispositif de détection de luminosité (9) présente une caméra (15) et la caméra (15) présente un objectif pourvu d'une distance focale réglée de manière fixe.

2. Éclairage opératoire (1) selon la revendication 1, **caractérisé en ce que** les luminaires individuels (3) sont divisés en plusieurs groupes de luminaires (11a, 11b), dans lequel plusieurs luminaires individuels (3a) affectés à un premier groupe de luminaires (11a) sont orientés et agencés les uns par rapport aux autres de telle manière que les axes longitudinaux (6) de leurs faisceaux lumineux (4) se coupent dans un premier plan focal (12) commun.

3. Éclairage opératoire (1) selon la revendication 2, **caractérisé en ce que** plusieurs luminaires individuels (3b) affectés à un deuxième groupe de luminaires (11b) sont orientés et agencés les uns par rapport aux autres de telle manière que les axes longitudinaux (6) de leurs faisceaux lumineux (4) se coupent dans un deuxième plan focal (13) commun situé à une certaine distance du premier plan focal (12).

4. Éclairage opératoire (1) selon les revendications 2 et 3, **caractérisé en ce que** l'unité de commande (10) est reliée électriquement aux luminaires individuels (3a) du premier groupe de luminaires (11a) et/ou aux luminaires individuels (3b) du deuxième groupe de luminaires (11b).

5. Éclairage opératoire (1) selon l'une quelconque des revendications 1 à 4, **caractérisé en ce que** le dispositif de détection de luminosité (9) comprend au moins un capteur de luminosité, qui présente au moins un phototransistor, au moins une photorésistance et/ou au moins une photodiode.

6. Éclairage opératoire (1) selon l'une quelconque des revendications 1 à 5, **caractérisé en ce que** le dispositif de détection de luminosité (9) présente au moins une zone de mesure (14) à l'intérieur du champ lumineux total (5), laquelle zone de mesure (14) présente une surface plus petite que celle du champ lumineux total (5) .

7. Éclairage opératoire (1) selon la revendication 6, **caractérisé en ce que** le dispositif de détection de luminosité (9) détermine la valeur de luminosité réelle de la zone de mesure (14) de façon globale ou partielle.

8. Éclairage opératoire (1) selon l'une quelconque des revendications 1 à 7, **caractérisé en ce qu'**une valeur de diaphragme de la caméra (15) est réglée ou peut être réglée fixement.

9. Éclairage opératoire (1) selon l'une quelconque des revendications 1 à 8, **caractérisé en ce qu'**une valeur de temps d'exposition de la caméra (15) est réglée ou peut être réglée fixement.

10. Éclairage opératoire (1) selon l'une quelconque des revendications 1 à 9, **caractérisé en ce que** le dispositif de détection de luminosité (9) comprend un appareil de mesure de luminance ou est réalisé en tant que tel.

11. Éclairage opératoire (1) selon l'une quelconque des revendications 1 à 10, **caractérisé en ce que** le dispositif de détection de luminosité (9) est inséré dans un corps de logement de luminaires (16) logeant les luminaires individuels (3) ou est intégré dans un dispositif de poignée (17) pouvant être relié au corps de logement de luminaires (19) de manière détachable.

12. Éclairage opératoire (1) selon l'une quelconque des revendications 1 à 11, **caractérisé en ce que** le dispositif de détection de luminosité (9) détecte la valeur de luminosité réelle de manière continue, par intervalles ou après la saisie manuelle d'une instruction d'enregistrement.

13. Procédé pour la commande d'un(1) destiné à éclairer un champ opératoire (2), dans lequel le scialytique (1)
- présente une pluralité de luminaires individuels (3) qui, dans un état allumé, forment chacun un faisceau lumineux (4) s'étendant le long d'un axe longitudinal (6) et produisant une zone de champ lumineux (8) dans un plan d'éclairage (7), dans lequel les zones de champ lumineux (8) des luminaires individuels (3) sont agencées dans le plan d'éclairage (7) les unes à côté des autres et/ou au moins en partie les unes au-dessus des autres de telle sorte qu'un champ lumineux total (5) est formé,
- un dispositif de détection de luminosité (9), qui est conçu pour déterminer une valeur de luminosité réelle dans le champ lumineux total (5), dans lequel le dispositif de détection de luminosité (9) présente une caméra (15) et la caméra (15) présente un objectif pourvu d'une distance focale réglée fixement, ainsi
- qu'une unité de commande (10) qui, en fonction de la valeur de luminosité réelle déterminée, agit sur les luminaires individuels (3) par une commande d'intensité d'éclairage,
dans lequel le dispositif de détection de luminosité (9) ainsi que l'unité de commande (10) sont conçus de telle manière et l'unité de commande (10) est reliée aux luminaires individuels (3) de telle manière que, en fonction de la valeur de luminosité réelle, une intensité d'éclairage d'un premier luminaire individuel (3a) peut être réglée de manière ciblée et indépendamment d'une intensité d'éclairage d'un deuxième luminaire individuel (3b) pour rendre plus claire ou plus sombre une première zone de champ lumineux (8a).
